# EUROPEAN PATENT SPECIFICATION

(11) **EP 2 590 693 B1**
(45) Date of publication and mention of the grant of the patent: **08.09.2021**
(21) Application number: 11831081.2
(22) Date of filing: 28.06.2011
(51) Int. Cl.: A61L 27/38, A61L 27/40, A61L 27/44, A61L 27/58, A61L 27/36, A61L 27/54

(54) **REINFORCED TISSUE GRAFT**
VERSTÄRKTES GEWEBETRANSPLANTAT
GREFFE DE TISSUS RENFORCÉE

(30) Priority: 28.06.2010 US 359067 P
(43) Date of publication of application: 15.05.2013
(73) Proprietor: THE CLEVELAND CLINIC FOUNDATION, Cleveland, OH 44195 (US)
(72) Inventor: DERWIN, Kathleen, A., Shaker Heights OH 44122 (US); IANNOTTI, Joseph, P., Strongsville OH 44136 (US); SAHOO, Sambit, Cleveland OH 44120 (US)
(74) Representative: FRKelly
(86) International application number: PCT/US2011/042138
(87) International publication number: WO 2012/047338

(56) References cited:
- WO-A2-2009/120966
- KR-A- 20060 041 726
- US-A1- 2006 029 639
- US-A1- 2007 260 268
- US-A1- 2009 306 688
- US-A1- 2010 028 396
- US-A1- 2010 028 396
- None

## Description

### TECHNICAL FIELD

This application is directed to tissue grafts and, in particular, is directed to a reinforced tissue graft.

### BACKGROUND

Researchers have experienced major challenges in designing methods to repair abdominal wall defects that result from traumatic injuries, surgical procedures (incisional hernias) or chronic diseases. Currently available synthetic and biological grafts have demonstrated only limited success. The graft should be biocompatible, have adequate mechanical properties and should also degrade at an appropriate rate to permit transition of function from the graft to native fascia. While the use of synthetic meshes has lowered incidence rates of incisional hernias to below 10%, these repairs are still frequently complicated by infection, visceral adhesion, extrusion and fistulation, and require re-operation. Biological grafts derived from decellularized tissues, such as dermis or small intestinal submucousa (SIS), have shown greater success than synthetics. They are better vascularized, less prone to be complicated by visceral adhesions, and can be used in the presence of contaminated or infected fields. While grafts derived from acellular dermis possess biomechanical properties that are similar to abdominal wall fascia, and have shown promise for hernia repair, they lose mechanical strength and integrity after implantation, resulting in laxity, stretching and poor suture retention, which in turn can lead to complications, such as bulging, dehiscence, and hernia recurrence. The dermis products are also commonly available in small sizes, and require pre-operative suturing of multiple pieces to repair large hernia defects.

WO2009120966 A2 discloses a biocompatible tissue graft including an extracellular matrix patch and a means for reinforcing the patch. US2009306688 A1 discloses a multilaminate or multiple layer implantable surgical graft comprising remodelable collagenous sheet material, the graft including one or more interweaving members to stitch together the graft to help prevent the layers from delaminating or separating during handling and the initial stages of remodeling.

US20100028396 A1 discloses laminated extracellular matrix scaffolds containing a polymer positioned between individual ECM sheets. The polymer may optionally contain bioactive molecules to enhance the functionality of the scaffold.

### SUMMARY

This application relates to a biocompatible tissue graft that includes a first layer of a bioremodelable collageneous material comprising extracellular matrix and a second layer of biocompatible synthetic remodelable, or substantially remodelable material attached to the first layer, wherein the second layer comprises a mesh of synthetic material. The graft further includes a plurality of first fibers and a plurality of second fibers stitched in a reinforcing pattern in the first layer and the second layer to mitigate tearing and/or improve fixation retention of the graft, and substantially maintain the improved properties while one or more layers of the graft remodels.

The first layer includes an extracellular matrix, and the second layer can include an extracellular matrix.

The fiber can include a natural or synthetic material and be stitched into the first layer and the second layer in a cross-hatched configuration. The first layer and the second layer are stitched together with the fiber. In some embodiments, the fiber can be selected from the group consisting of collagen, silk, sericin free silk, modified silk fibroins, polyesters like PGA, PLA, polylactic-co-glycolic acid (PLGA), polyethyleneglycol (PEG), polyhydroxyalkanoates (PHA), polyethylene terephthalate (PET), polyethylene (PE), ultra-high molecular weight polyethylene (UHMWPE), blends thereof, and copolymers thereof.

In an aspect of the application, the first layer and, optionally, the second layer can include extracellular matrix that is decellularized. The first layer and/or the second layer can also be seeded with at least one differentiated cell or progenitor cell. The first layer and/or the second layer can further include at least one biologically active molecule selected from the group consisting of drugs, sclerosing agents, enzymes, hormones, cytokines, colony-stimulating factors, vaccine antigens, antibodies, clotting factors, angiogenesis factors, regulatory proteins, transcription factors, receptors, structural proteins, nucleic acid therapeutic agents, such as plasmids, vectors, siRNA, and micro-RNA, and combinations thereof.

This application also discloses to a method of constructing a biocompatible tissue graft. The method includes providing an extracellular matrix layer, providing a synthetic layer, attaching the extracellular matrix layer to the synthetic layer, and stitching a plurality of first and second fibers into the layers in a cross-hatched reinforcement pattern to mitigate tearing and/or improve fixation retention of the graft, and substantially maintain the improved properties while one or more layers of the graft remodels.

The method can further include providing a second extracellular matrix layer and attaching the second extracellular layer to the first extracellular layer and/or the synthetic layer. The fiber can be stitched into the extracellular matrix layers and the synthetic layer in a reinforcement pattern to mitigate tearing and/or improve fixation retention of the graft, and substantially maintain the improved properties while one or more layers of the graft remodels.

In an aspect of the application, the second extracellular matrix layer is provided only at one end of the tissue graft.

The fiber can include a natural or synthetic material and is stitched into at least one of the first extracellular matrix layer, the second extracellular matrix layer, and/or the synthetic layer in, for example, a cross-hatched configuration. The first extracellular matrix layer, the second extracellular layer, and/or the synthetic layer can also be stitched together with the fiber.

In another aspect of the application, the graft can have a multilayer construction and include a first layer, a second layer, and a third layer. The first layer, the second layer, or the third layer can comprise at least one layer of an extracellular matrix and at least one layer of a biocompatible substantially remodelable synthetic material or biocompatible non-remodelable synthetic material. The first layer, the second layer, and/or third layer can be stitched together with a fiber. The first layer and the second layer are stitched together with the fiber. In an example, the first layer, the second layer, and the third layer can be stitched together with the fiber. The fiber can be stitched into the extracellular matrix layer(s) and optionally the synthetic layer(s) in a reinforcement pattern to mitigate tearing and/or improve fixation retention of the graft, and substantially maintain the improved properties while one or more layers of the graft remodels.

In some embodiments, the fiber can be selected from the group consisting of collagen, silk, sericin free silk, modified silk fibroins, polyesters like PGA, PLA, polylactic-co-glycolic acid (PLGA), polyethyleneglycol (PEG), polyhydroxyalkanoates (PHA), polyethylene terephthalate (PET), polyethylene (PE), ultra-high molecular weight polyethylene (UHMWPE), blends thereof, and copolymers thereof.

### BRIEF DESCRIPTION OF THE DRAWINGS

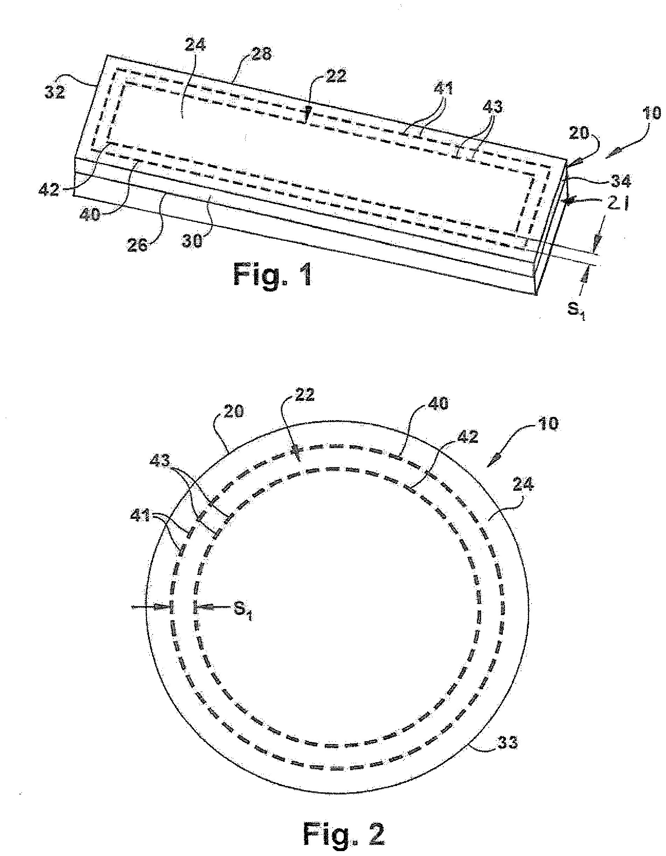

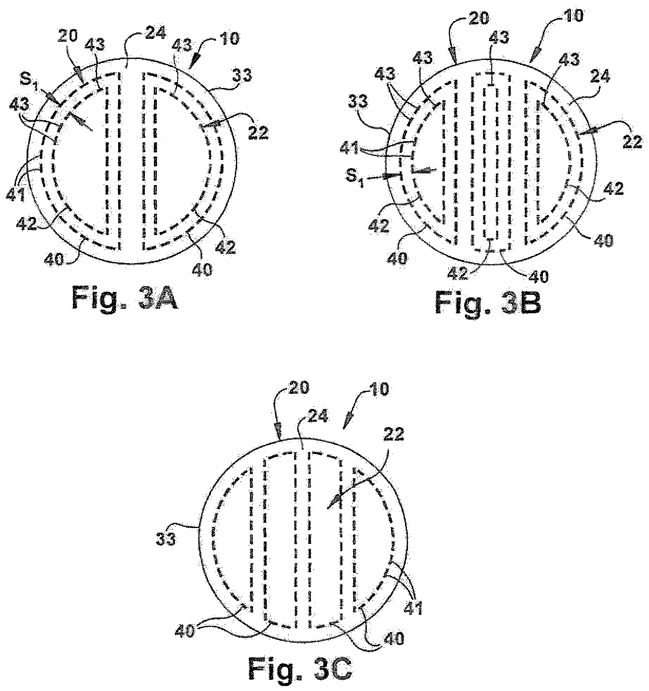

The foregoing and other features and advantages of the application will become apparent to those skilled in the art to which the present invention relates upon reading the following description with reference to the accompanying drawings, in which:
Reference Fig. 1 is schematic illustration of a tissue graft having reinforcement means;
Reference Fig. 2 is a top view of a tissue graft having a reinforcement means;
Reference Fig. 3A is a top view of a tissue graft having a reinforcement means;
Reference Fig. 3B is a top view of a tissue graft having a reinforcement means;
Reference Fig. 3C is a top view of a tissue graft having a reinforcement means;
Fig. 4A is a schematic illustration of a tissue graft having a reinforcement means in accordance with an embodiment of the present invention;
Fig. 4B is a top view of a tissue graft having a reinforcement means in accordance with yet another embodiment of the present invention;
Figs. 5-8 are schematic illustrations of tissue grafts having multiple layers in accordance with yet another embodiment of the present invention;
Fig. 9 is graph illustrating average load-displacement curves of dermis, synthetic mesh, dermis layered against mesh and stitched together using 6PLA/2PGA polymer braids; and
Fig. 10 is a graph illustrating increased suture retention load of dermis and mesh layers stitched together with 6PLA/2PGA polymer braids, compared to dermis or synthetic mesh alone or dermis and mesh layers unstitched.

### DETAILED DESCRIPTION

The present application is directed to biocompatible tissue grafts and, in particular, is directed to a fiber reinforced biocompatible tissue graft with improved fixation retention properties. The tissue graft can be used to treat a tissue defect of a subject (e.g., human being), such as a musculoskeletal defect, or in tendon-to-bone repairs (e.g., rotator cuff injury) or soft-tissue repairs, such as the repair of lacerated muscles, muscle transfers, abdominal wall reconstruction, hernia repair, repair of compartment syndrome releases, or used in tendon reinforcement. The tissue graft may also be used as a bridging material in a subject, such as where the gap between a tendon and the associated bone is too large to repair conventionally or in abdominal wall procedures with loss of tissue domain.

The biocompatible tissue graft can be used as an overlay (over or at the interface of the repair), an interpositional (to fill a space between a tissue and its attachment), or an underlay (under or at the interface of the repair). In one example, the tissue graft can be applied over the bone-tendon repair site and fixed to the bone and tendon to augment the repair. In another example, the tissue graft can be used to repair an abdominal wall injury site or abdominal wall hernia. Abdominal hernias can be repaired by onlay, inlay, or underlay techniques, where the graft is placed over, within, or under the defect in the abdominal wall muscle. The underlay technique involves placing the tissue graft beneath the abdominal musculature (underlying the hernia defect and extending beyond it), and anchoring the edges of the graft to the muscle using sutures.

In accordance with an embodiment of the application, the biocompatible tissue graft includes a first layer of bioremodelable collagenous material comprising extracellular matrix, a second layer of biocompatible synthetic remodelable, or substantially remodelable material attached to the first layer, wherein the second layer comprises a mesh of synthetic material and a plurality of first fibers and a plurality of second fibers stitched in a cross-hatched reinforcing pattern in the first layer and second layer to mitigate tearing of the graft, improve fixation retention properties of the graft, and substantially maintain the improved properties while one or more layers of the graft remodels. The plurality of first and second fibers also attach the first layer to the second layer.

The term "biocompatible" as used herein refers to a material that is substantially non-toxic in the *in vivo* environment of its intended use, and that is not substantially rejected by the patient's physiological system *(i.e.,* is non-antigenic). This can be gauged by the ability of a material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1. Typically, these tests measure a material's toxicity, infectivity, pyrogenicity, irritation potential, reactivity, hemolytic activity, carcinogenicity and/or immunogenicity. A biocompatible material, when introduced into a majority of patients, will not cause an undesirably adverse, long-lived or escalating biological reaction or response, and is distinguished from a mild, transient inflammation, which typically accompanies surgery or implantation of foreign objects into a living organism.

The terms "remodelable" and "bioremodelable" as used herein refer to the ability of the material to be resorbed by the host and replaced by host tissue, *i.e.,* remodeled from one material to another. Remodeling can occur in various microenvironments within a body, including without limitation soft tissue, a sphincter muscle region, body wall, tendon, ligament, bone and cardiovascular tissues. Upon implantation of a remodelable material, cellular infiltration and neovascularization are typically observed over a period of about 5 days to about 6 months or longer, as the remodelable material acts as a matrix for the ingrowth of adjacent tissue with site-specific structural and functional properties. The remodelable material may be completely or partially resorbed and replaced by the host during this time period.

The term, "substantially remodelable" materials as used herein include materials that are unlikely to be completely resorbed *and rep*laced by host tissue. These materials can permit tissue ingrowth at a much slower rate than the rate of tissue growth in the remodelable material. Tissue growth through the substantially remodelable material is typically only observable after sufficient periods of implantation in a body vessel that permit substantial amounts of tissue growth in the remodelable material.

By substantially maintaining "the improved properties while one or more layers of the graft remodels", it is meant that the improved fixation properties, such as suture retention properties, are maintained or minimally decreased after implantation of the graft *in vivo* due to, for example, collagenase digestion of the remodelable or substantially remodelable layers, while the remodelable or substantially remodelable layers of the graft are resorbed by the host and replaced by host tissue and until the host tissues have sufficient strength.

The bioremodelable collagenous material used to form the first layer comprises extracellular matrix (ECM). In some embodiments, the bioremodelable collagenous material used to form the first layer can include at least one sheet of extracellular matrix (ECM). The ECM can be derived from any mammalian ECM, *such as* fascia, and in particular, fascia lata from humans. The ECM can be derived from other connective tissue materials, such as dermis (skin) as long as the ECM is biocompatible with the target site or the tissue injury being treated in the subject or both. The ECM can also be derived, for example, from other tissues and/or other materials, such as bone, articular cartilage, meniscus, myocardium, pericardium, periosteum, artery, vein, stomach, large intestine, small intestine (such as small intestine submucosa), peritoneum, mesothelium, diaphragm, tendon, ligament, neural tissue, meninges, striated muscle, smooth muscle, bladder, ureter, abdominal wall fascia, and combinations thereof.

In another embodiment of the application, the bioremodelable collagenous material used to form the first layer can be ECM obtained directly from mammalian tissue (such as an autograft, allograft or xenograft). These tissues may be obtained from patients at the time of surgery or a commercial source, such as a tissue bank medical device company. ECM obtained from tissue banks and other commercial sources may be formed using proprietary processing techniques or modified by additional processing techniques before it is used. In one example, these techniques can be used to remove cells (e.g., decellularized) and other potentially infectious agents from the ECM.

In other embodiments, the bioremodelable collagenous material used to form the first layer can comprise a collagenous sheet that is derived from a mammalian source or is plant-based. For example, the collagenous sheet may constitute collagen that is derived from the peritoneum of pigs (Kensey Nash ECM Surgical Patch manufactured by Kensey Nash Corporation of Exton, PA). In another example, the collagenous sheet may constitute collagen that is derived from the Procollagen of plants and sold under the trade name COLLAGE RH (manufactured by CollPlant of Ness-Ziona, Israel). The COLLAGE RH may be formed by collecting Procollagen from plants and processing it into pure, fibrin forming collagen.

The second layer is formed from a biocompatible synthetic remodelable or substantially remodelable material. In an aspect of the application, the second layer when attached to the first layer can structurally reinforce and/or mechanically enhance the first layer and the tissue graft. For example, the second layer can exhibit a construction that facilitates retention of the reinforcing means in the tissue graft and thereby helps to enhance and/or substantially maintain suture retention as the first layer remodels and prevents relative movement between the first layer and the second layer.

In some embodiments described herein, the second layer can include a two-dimensional or three-dimensional fibrous matrix or mesh construct, which may be shaped or formed for the particular application.

Typically, the mesh is a pliable material, such that it has sufficient flexibility to be wrapped around the external surface of a body passageway or cavity, or a portion thereof. The mesh may be capable of providing support to the graft. In certain aspects, the mesh may be adapted to release an amount of a therapeutic agent.

Mesh materials may take a variety of forms. For example, the mesh may be in a woven, knit, or non-woven form and may include fibers or filaments that are randomly oriented relative to each other or that are arranged in an ordered array or pattern. In one embodiment, the mesh may be in the form of a fabric, such as a knitted, braided, crocheted, woven, non-woven (e.g., a melt-blown, wet-laid, or electrospun) or webbed fabric. In one embodiment, the mesh may include a natural or synthetic biodegradable polymer that may be formed into a knit mesh, a weave mesh, a sprayed mesh, a web mesh, a braided mesh, a looped mesh, and the like. Preferably, the mesh has intertwined threads that form a porous structure, which may be, for example, knitted, woven, or webbed.

The structure and properties of the mesh used in the graft can depend on the application and the desired mechanical *(i.e.,* flexibility, tensile strength, and elasticity) and degradation properties, and the desired loading and release characteristics for selected therapeutic agent(s). The mesh should have mechanical properties such that the graft can remain sufficiently strong until the surrounding tissue has healed. Factors that affect the flexibility and mechanical strength of the mesh include, for example, the porosity, fabric thickness, fiber diameter, polymer composition (e.g., type of monomers and initiators), process conditions, and the additives that are used to prepare the material.

Typically, the mesh possesses sufficient porosity to permit the flow of fluids through the pores of the fiber network and to facilitate tissue ingrowth. Generally, the interstices of the mesh should be wide enough apart to allow light visible by eye, or fluids, to pass through the pores. However, materials having a more compact structure also may be used. The flow of fluid through the interstices of the mesh may depend on a variety of factors, including, for example, the stitch count or thread density. The porosity of the mesh may be further tailored by, for example, filling the interstices of the mesh with another material *(e.g.,* particles or polymer) or by processing the mesh *(e.g.,* by heating) in order to reduce the pore size and to create non-fibrous areas. Fluid flow through the mesh can vary depending on the properties of the fluid, such as viscosity, hydrophilicity/hydrophobicity, ionic concentration, temperature, elasticity, pseudoplasticity, particulate content, and the like. In one example, the interstices of the mesh can be large enough so as to not prevent the release of impregnated or coated therapeutic agent(s) from the mesh, and the interstices preferably do not prevent the exchange of tissue fluid at the application site.

Mesh materials can also be sufficiently flexible so as to be capable of conforming to the shape of an anatomotical surface and/or the first layer. In certain cases, the mesh material may be sufficiently flexible so as to be capable of being wrapped around all or a portion of the first layer and/or the external surface of a body passageway or cavity. Flexible mesh materials are typically in the form of flexible woven or knitted sheets having a thickness ranging from about 25 microns to about 3000 microns; preferably from about 50 to about 1000 microns. Mesh materials for use in the practice of the invention typically range from about 100 to 400 microns in thickness.

In some embodiments, the second layer may be formed of any one or combination of known biocompatible, synthetic materials, including polypropylene (PP), polyurethane (PU), expanded polytetrafluoroethylene (ePTFE), polylactic acid (PLA), polyglycolic acid (PGA), polycaprolactone (PCL), polydioxanone (PDO), trimethylene carbonate (TMC), polyvinyl alcohol (PVA), poly(ethylene oxide), poly(acrylic acid), poly(vinyl alcohol), poly(N-isopropyl acrylamide), poly(vinyl pyrrolidone) (PVP), poly (methacrylic acid), poly(p-styrene carboxylic acid), poly(p-styrenesulfonic acid), poly(vinylsulfonicacid), poly(ethyleneimine), poly(vinylamine), poly(anhydride), poly(HEMA), polyhydroxybutyrate (PHB), . These compositions include copolymers of the above polymers as well as blends and combinations of the above polymers.

In one example, the second layer is constructed of a medical-grade polypropylene mesh fabric commercially available from ATEX technologies, Inc. (Pinebluff, N.C.). In another example, the second layer is constructed of a polylactic acid mesh (X-Repair) available from Synthasome, Inc. (San Diego, CA).

The fiber stitched into the first layer and the second layer can be capable of not only attaching or securing the first layer to the second layer, but also mitigating tearing of the graft when the graft is implanted and loaded at the repair site and/or increasing or improving the fixation retention properties of the tissue graft beyond that which is present in a layer of the ECM alone. The fixation retention properties can be tailored to increase the graft's ability to remain secured to anatomic structures, such as bone and soft tissues, when used to treat a tissue defect.

The graft may be secured to these anatomical structures by, for example, weaving, screws, staples, tacks, sutures, pins, rods, other mechanical or chemical fastening means including tissue adhesives/glues, such as fibrin sealant, frog glue, and cyanoacrylate glue, or combinations thereof. For instance, the graft may be secured to the treated tissue via different suture configurations, such as massive cuff, mattress and simple suture and different fixation techniques, such as Synthes screw or Biotenodesis screw fixation or suture anchors with a Krakow stitch.

The fiber is stitched in a reinforcement pattern that can increase the fixation retention of the tissue graft, which will result in a tissue graft having improved mechanical properties for implantation and repair of anatomical defects in a subject. The reinforcement pattern mitigates tearing and/or improves the fixation retention properties of the tissue graft when administered to a subject being treated. The stitch pattern is a cross-hatched stitch pattern.

The fiber can enhance the fixation retention of the tissue graft once stitched into the graft and substantially maintain the fixation retention properties while one or more layers of the graft remodels. The fiber can be formed from a biocompatible material that is bioresorbable, biodegradable, or non-resorbable.

The fiber can be made from natural and/or synthetic materials so long as the material is biocompatible. One example of a biocompatible material that can be used to form the fiber is silk. The silk may include, for example, sericin-free silk fibroin or silk-fibroin modified with a peptide sequence that sequesters growth factors *in vivo,* such as disclosed in U.S. Patent No. 6,902,932, which is herein incorporated by reference. The fibers can also be formed from biodegradable polymers including PGA, PLA, poly(lactic-co-glycolic acid) (PLGA), poly(ethylene glycol) (PEG), blends thereof, and copolymers thereof. By way of example, the reinforcing fiber may include a core of PGA surrounded by a sheath of reinforced PLA fibers. The PGA and PLA may be obtained, for example, from Concordia Fibers in Coventry, RI. Other examples of biocompatible polymers that can be used to form the fiber are resorbable polyesters, such as polyhydroxyalkanoates (PHA), and non-resorbable fibers, such as polyethylene terephthalate (PET) and ultra-high molecular weight polyethylene (UHMWPE). The biocompatible fibers can also be formed from mammalian collagen or plant-based collagen such as COLLAGE RH.

It will be appreciated that the biocompatible fiber can be formed from other biocompatible materials, such as other biocompatible materials that are typically used in forming biocompatible fibers for *in vivo* medical applications.

In some embodiments, the fiber can exhibit a high modulus of elasticity and a failure load tailored to meet particular design criterion corresponding with *in vivo* strength requirements of the treated tissue. For example, fiber reinforced layers used for the treatment of large and massive rotator cuff tears should exhibit failure loads of greater than about 250 Newtons (N) at the time of implantation, and substantially maintain these properties after implantation *in vivo* until the host tissues have sufficient strength. Alternatively, fiber reinforced layers used for the treatment of tissues experiencing lower natural loads may be required to exhibit failure loads of about 30 N to about 50 N. For example, fiber reinforced layers used for the treatment of ventral abdominal wall defects and hernias should exhibit burst strengths greater than 50 N/cm, suture retention strengths greater than 20 N, tear resistance greater than 20 N, and strain at 16 N/cm load between about 10% and about 30%, at the time of implantation, and substantially maintain these properties after implantation *in vivo* until the host tissues have sufficient strength. It will be understood, however, that the fibers, their stitch design (i.e., reinforcement pattern), or the particular biomaterial layers can be tailored to produce failure loads of the reinforced construct commensurate in scale to any tissue treated within the body.

In still other embodiments, at least one of the fibers, the first layer, and the second layer (where applicable) can be mechanically, chemically or biologically modified to enhance adhesion between the fibers, the first layer, and the second layer to further secure the first layer to the second layer and/or fibers to the first layer and the second layer. This modification may occur before or after attachment of the first layer to the second layer as well as before or after the fibers are incorporated into the first layer and the second layer. This modification may be performed on a portion of or substantially all of the stitched fibers or the first layer or the second layer or all three. During loading of the tissue graft, the fibers may begin to displace relative to the first layer and/or the second layer and may ultimately completely slip out from the first layer and the second layer and become the primary load bearing components of the reinforced tissue construct. It therefore becomes desirable to mitigate or prevent fiber slippage in order to ensure that usage loads are borne by the entire graft and not just the fibers. Adhesion characteristics of the fibers can be improved by ablation via ultra-violet (UV) or infrared (IR) light, UV cross-linking or chemical cross-linking, plasma etching, ion etching, coating the fibers with microspheres, application of adhesives, such as biocompatible synthetic adhesives, bioadhesives, or combinations thereof. These treatments can likewise be performed on the first layer and the second layer.

In another embodiment, the first layer and/or the second layer can be seeded with a single type or a plurality of differentiated cells or progenitor cells that become dispersed in the first layer and/or second layer. Examples of differentiated cells are fibroblasts, chondrocytes, osteoblasts, tenocytes, skeletal muscle cells, smooth muscle cells, endothelial cells, and neural cells. Examples of progenitor cells are bone marrow-derived progenitor cells, adipose derived stem cells, hematopoietic stem cells, endothelial progenitor cells, mesenchymal stem cells, multipotent adult progenitor cells (MAPCs), embryonic stem cells, stromal cells, and induced pluripotent stem cells. The differentiated and progenitor cells can be autologous, allogeneic, xenogeneic or a combination thereof. The differentiated and progenitor cells can also be genetically modified. Genetically modified cells can include cells that are transfected with an exogenous nucleic acid and that express a polypeptide of interest including, for example, a growth factor, a transcription factor, a cytokine, and/or a recombinant protein.

The first layer and/or second layer can additionally or optionally include at least one biologically active molecule dispersed or seeded therein. Any desired biologically active molecule can be selected for impregnating into the first layer and/or the second layer. For example, the biologically active molecule can include drugs, such as antibiotics, sclerosing agents, enzymes, hormones, cytokines, colony-stimulating factors, vaccine antigens, antibodies, clotting factors, angiogenesis factors, regulatory proteins, transcription factors, receptors, structural proteins, nucleic acid therapeutic agents, such as plasmids, vectors, siRNA, and micro-RNA, and combinations thereof. The biologically active molecule can be chosen based on where the graft is to be located in the subject or the physiological requirements of the subject or both. For example, if the graft is used to repair a tendon, the biologically active molecule which is seeded on or into the first layer and the second layer can be a growth factor, such as IGF-I, TGF-β, VEGF, bFGF, BMP or combinations thereof.

Optionally, a high-molecular weight (e.g., greater than about 250 kDa) hyaluronic acid (HA) can be incorporated into the tissue graft prior to, during, or after stitching of the fibers into the first layer and the second layer. When incorporated into the tissue graft, HA can potentially inhibit the migration of inflammatory cells, induce the migration of non-inflammatory cells, and promote angiogenesis, which would promote integration of the tissue graft with the underlying host tissues.

The high-molecular weight HA can be cross-linked within the first layer and/or the second layer to mitigate diffusion of the HA from the first layer and/or second layer. Cross-linked, high-molecular-weight HA can be retained in the first layer and/or second layer for extended periods *in vitro.* An example of a cross-linked HA material that can be used in this application is prepared by substituting tyramine moieties onto the HA chains and then linking tyramines to form dityramine linkages between HA chains, effectively cross-linking or gelling the HA into the first layer and/or the second layer. Examples of dityramine-cross-linked HA composition and chemistry are disclosed in U.S. Patent No. 6,982,298 and U.S. Application Publications Nos. 2004/0147673, 2005/0265959, and 2006/0084759, which are herein incorporated by reference in their entirety. The tyramine-substitution rate on the HA molecules may be about five percent based on available substitution sites as disclosed in the aforementioned publications.

TS-HA can be impregnated into the first layer and/or the second layer, and then immobilized within the first layer and/or second layer by cross-linking of the tyramine adducts to form dityramine linkages, thereby producing a cross-linked HA macromolecular network. The TS-HA can be impregnated into the first layer and/or second layer prior to or after stitching the first layer and/or second layer. The TS-HA can be used to attach fibronectin functional domains (FNfds) to the first layer and/or second layer in order to further promote healing, cell migration, and anti-inflammatory capabilities. FNfds possess the ability to bind essential growth factors that influence cell recruitment and proliferation *(e.g.,* PDGF-BB and bFGF). The FNfds may, for example, constitute fibronectin peptide "P-12" with a C-terminal tyrosine to allow it to be cross-linked to TS-HA.

Optionally, tyramine-substituted gelatin (TS-gelatin) can be immobilized within and on the surface of fascia ECM by cross-linking of the tyramine adducts to form dityramine bridges. The TS-gelatin can elicit a host macrophage response and thus promote cellular infiltration and scaffold integration. (See for example, U.S. Patent No. 6,982,298 and U.S. Patent Application Publication No. 2009/0252700, both of which are incorporate by reference in their entirety).

One non-claimed example of a tissue graft is illustrated in reference Fig. 1. The tissue graft 10 includes first and second layers 20 and 21 of biocompatible material. Each of the first layer 20 and the second layer 21 can include a bioremodelable collagenous material, *e.g.,* an ECM layer, or a substantially remodelable or non-remodelable synthetic or natural material. Reinforcing means 22 are provided in the first and second layers 20 and 21 in a reinforcing pattern.

The tissue graft 10 is illustrated as having a generally rectangular strip shape *(e.g.,* about 5 cm long by about 2 cm wide) although the graft 10 can have other shapes, such as an elliptical shape, a circular shape, a square shape, etc. *(e.g.,* Figs 2, 3, 4). The graft 10 includes a top surface 24 and a substantially parallel bottom surface 26 spaced from the top surface. A first side 28 and second side 30 connect the top surface 24 to the bottom surface 26. The first and second sides 28, 30 extend generally parallel to one another. The graft 10 further includes a front surface 32 and rear surface 34 which connect the first side 28 to the second side 30. The front and rear surfaces 32, 34 extend generally parallel to one another.

The reinforcing means 22 can include at least one fiber disposed or provided within the first layer 20 and/or the second layer 21 by, for example, conventional stitching techniques. By stitching, it is meant that at least one fiber of the reinforcing means 22 is stitched into the first layer 20 and/or second layer 21 such that each stitch of the reinforcing means extends between and through both the top surface 24 and the bottom surface 26 of the first layer 20 and/or second layer 21 to securely fasten the reinforcing means to the first layer 20 and/or second layer 21.

The reinforcing means 22 may exhibit any reinforcement configuration or pattern that increases the fixation properties of the graft 10. One such configuration is illustrated in Fig. 1 in which first and second fibers 40, 42 are stitched into the first layer 20 and second layer 21 in geometrically concentric configurations. Additionally or alternatively, the stitch lines of the fibers can be placed further away from the edges of the graft 10 to delay, mitigate, or prevent slipping of the fibers 40, 42 within the first layer 20 and second layer 21. Although Fig. 1 illustrates two fibers in a geometrically concentric pattern, it will be understood that more or fewer fibers can be stitched into the first layer 20 and second layer 21 in a geometrically concentric pattern. Additionally, it will be appreciated that additional fibers can be stitched into the first layer 20 and/or second layer 21 in other reinforcement patterns.

As shown in reference Fig. 1, the first fiber 40 can extend substantially parallel to, and be spaced inwardly from, the periphery of the graft 10. By way of example, the first fiber 40 can extend substantially parallel to the first and second sides 28, 30 and the front and rear surfaces 32, 34 of the graft 10 such that the first fiber 40 exhibits a generally rectangular configuration. The first fiber 40 can comprise a plurality of interconnected stitches 41. The ends of the fiber 40 may be stitched together (not shown) to form a continuous stitching construction.

The second fiber 42 can extend substantially parallel to the first fiber 40 and be disposed radially inward of the first fiber 40 within the graft 10. In this configuration, the first and second fibers 40, 42 form a generally geometrically concentric construction in a peripheral double pass orientation. The second fiber 42 can comprise a plurality of interconnected stitches 43. The second fiber can be substantially uniformly spaced inward from the first fiber 40 by a gap indicated by "s₁". The gap s₁ may be, for example, on the order of about 1 mm to about 3 mm (e.g., about 2 mm), although other spacing configurations will be understood. It will be appreciated that although the gap s₁ between the fibers 40 and 42 is substantially uniform, the gap s₁ may vary depending on reinforcement pattern in which the fibers 40 and 42 are stitched. The ends of the second fiber 42, like first fiber 40, may be stitched together (not shown) to form a continuous stitching construction.

The first fiber 40 and the second fiber 42 can be stitched in the graft so that the number of stitches per inch is, for example, about 10 stitches per inch to about 20 stitches per inch *(e.g.,* about 15 stitches per inch). Generally, the more stitches per inch, the greater the strength of the reinforcing means 22 and the fixation retention properties of the tissue graft 10. In some examples, however, it may be desirable to use less stitches per inch to avoid excessive needle penetrations in the first layer 20 and/or second layer 21, which may potentially weaken the tissue graft 10.

Other non-claimed examples of concentric reinforcement stitch patterns or configurations are illustrated in reference Fig. 2 and reference Figs. 3A-3C. The configurations in Fig. 2 and Figs. 3A-3C are similar to the configuration of Fig. 1, except that in Fig. 2 the graft 10 is substantially circular and therefore the reinforcing means 22 is provided in the graft 10 in a generally circular configuration or orientation. Fig. 2 illustrates one example of a graft 10 that includes generally concentric reinforcement means 22 in a peripheral double pass orientation. The reinforcement means 22 includes a first fiber 40 that comprises a plurality of interconnected stitches 41 and a second fiber 42 that comprises a plurality of interconnected stitches 43. The first fiber 40 can extend substantially parallel to, and be spaced inwardly from, a peripheral surface 33 of the graft 10 such that the first fiber has a generally circular configuration. The second fiber 42 can extend substantially parallel to the first fiber 40 and be disposed radially inward of the first fiber within the graft 10. In this configuration, the first and second fibers 40, 42 form a generally concentric construction.

Fig. 3A illustrates another non-claimed example of the reinforcing means 22 comprising two concentric patterns 43. Each concentric pattern 43 includes a first strand 40 that comprises a plurality of interconnected stitches 41 and a second strand 42 that comprises a plurality of interconnected stitches 43. Each first fiber 40 can extend substantially parallel to, and be spaced inwardly from, a peripheral surface 33 of the graft 10 such that each first fiber has a generally circular configuration. Each second fiber 42 can extend substantially parallel to the first fiber 40 and be disposed radially inward of the first fiber within the graft 10. In this configuration, each pair of first and second fibers 40, 42 form a generally concentric construction. Although the two concentric patterns 43 are illustrated as being substantially semi-circular, it will be understood that each concentric pattern may exhibit alternative constructions such as, for example, rectangular (e.g., in a two rectangle double pass orientation), elliptical, triangular or combinations thereof.

Fig. 3B illustrates another non-claimed example of the reinforcing means 22 comprising three concentric patterns 43. Each concentric pattern 43 comprises a first strand 40 comprising a plurality of interconnected stitches 41 and a second strand 42 comprising a plurality of interconnected stitches 43. Each first fiber 40 can extend substantially parallel to, and be spaced inwardly from, a peripheral surface 33 of the graft 10 such that each first fiber has a generally circular configuration. Each second fiber 42 can extend substantially parallel to the first fiber 40 and be disposed radially inward of the first fiber within the graft 10. In this configuration, each pair of first and second fibers 40, 42 form a generally concentric construction. It will be understood that each concentric pattern may exhibit any constructions such as, for example, rectangular (e.g., in a three rectangle double pass orientation), elliptical, triangular, semi-circular, circular or combinations thereof.

Fig. 3C illustrates yet another non-claimed example of a reinforcing means 22 that includes a plurality of first strands 40, which comprise a plurality of interconnected stitches 41 but without or free of concentric second strands 42. In particular, the first strands 40 may comprise four substantially parallel and elliptical discrete first strands. Although the four first strands 40 are illustrated as being substantially elliptical, it will be understood that each first strands may exhibit alternative constructions such as, for example, rectangular (e.g., in a four rectangle single pass orientation), semi-circular, circular, triangular or combinations thereof. It will also be understood that one or more of the first strands could have a geometrically concentric pattern with a second strand.

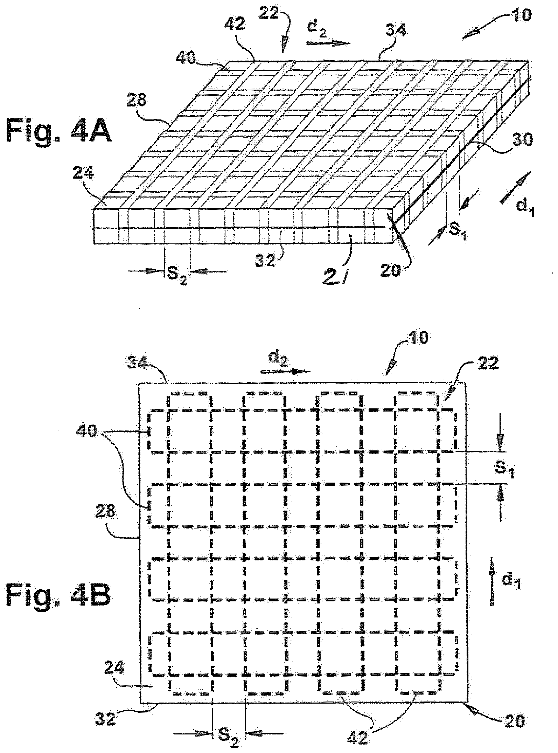

Fig. 4A is a schematic illustration of a tissue graft 10 that includes a first layer 20, second layer 21, and a reinforcing means 22 in accordance with an example of the graft 10 in accordance with the invention. The reinforcing means 22 includes a plurality of first fibers 40 and a plurality of second fibers 42 stitched in a cross-hatched pattern across the graft 10 and between the first and second sides 28, 30 and the front and rear surfaces 32, 34. Although Fig. 4A illustrates six first fibers 40 and eight second fibers 42, it is understood that more or less of each fiber may be utilized in accordance with the present invention. The first fibers 40 can extend in a first direction, indicated by "d₁", across the top surface 24 of the graft 10 from the first side 28 to the second side 30. Each of the first fibers 40 can extend parallel to one another and be spaced apart by a gap indicated by "s₁". The gap s₁ may be, for example, on the order of about 1 mm to about 3 mm, although other spacing configurations will be understood. The gap s₁ may be uniform or may vary between first fibers 40.

The second fibers 42 can extend in a second direction, indicated by "d₂", across the top surface 24 of the graft 10 from the front surface 32 to the rear surface 34. The directions "d₁" and "d₂" in which the first and second fibers 40, 42 extend may be configured such that the first fibers and the second fibers are oriented perpendicular to each other. Each of the second fibers 42 can extend parallel to one another and be spaced apart by a gap indicated by "s₂". The gap s₂ may be, for example, on the order of about 1 mm to about 3 mm, although the gap can have other spacing configurations. The gap s₂ may be uniform or may vary between second fibers 42. The second fibers 42 are disposed in an overlying fashion relative to the first fibers 40 such that the first fibers are disposed between the top surface 24 of the graft 10 and the second fibers. The second fibers 42, however, could alternatively be disposed between the top surface 24 of the graft 10 and the first fibers 40.

Fig. 4B illustrates that the reinforcing means 22 comprises a plurality of first fibers 40 and a plurality of second fibers 42 stitched in a cross-hatched pattern across the graft 10 and between the first and second sides 28, 30 and the front and rear surface 32, 34. Although Fig. 4B illustrates four first fibers 40 and four second fibers 42, it is understood that more or less of each fiber may be utilized in accordance with the present invention. Each of the first fibers 40 extends from the first side 28 to the second side 30 of the graft 10. Each of the second fibers 42 extends from the front surface 32 to the rear surface 34 of the layer 20. The ends of the first fibers 40 and the ends of the second fibers 42, respectively, are stitched together (not shown) to form a continuous stitching construction.

The second fibers 42 are disposed in an overlying fashion relative to the first fibers 40 such that the first fibers are disposed between the top surface 24 of the graft 10 and the second fibers. The second fibers 42, however, could alternatively be disposed between the top surface 24 of the graft 10 and the first fibers 40. Although the first and second fibers 40, 42 are illustrated as having a substantially rectangular shape *(e.g.,* a rectangular cross-hatch orientation), it will be understood that the first fiber 40 and/or the second fiber 42 may exhibit alternative constructions such as elliptical, semi-circular, circular, triangular or combinations thereof.

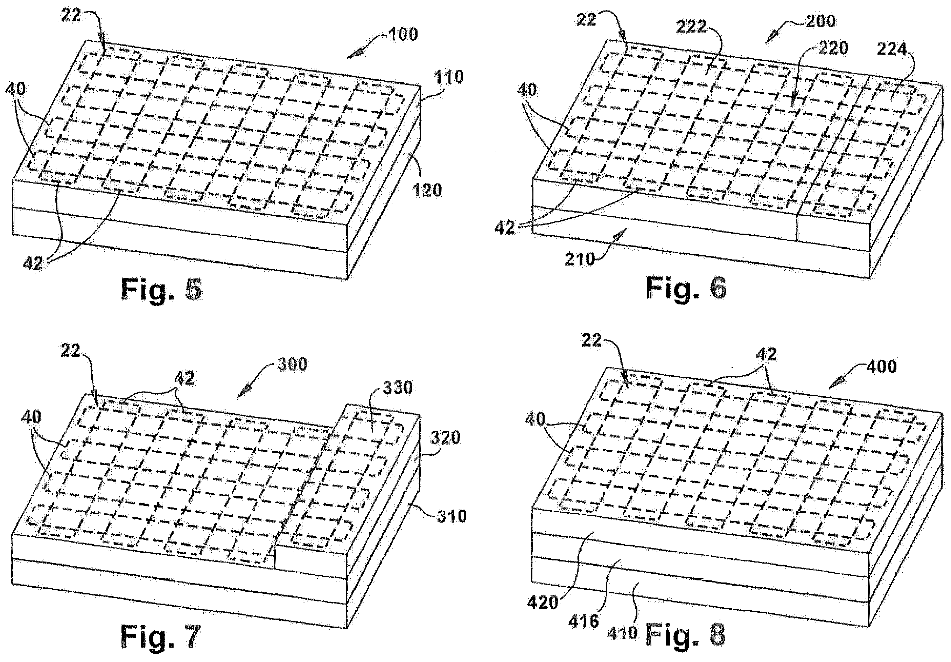

Figs. 5-8 illustrate various tissue graft constructions 100, 200, 300, 400 having multiple layers in accordance with another aspect of the present invention. In Fig. 5, the tissue graft 100 includes first and second layers 110, 120 of biocompatible material. Each of the first layer 110 and the second layer 120 can include a bioremodelable collagenous material, e.g., an ECM layer, or a substantially remodelable or non-remodelable synthetic or natural material. Reinforcing means 22 are provided in the first and second layers 110, 120 in a reinforcing pattern as previously described. As shown in Fig. 5, the reinforcing means 22 comprises a plurality of first fibers 40 and a plurality of second fibers 42 stitched in a cross-hatched pattern across and through both layers 110, 120 in order to mitigate tearing and/or improve fixation retention of the first and second layers.

In Fig. 6, the tissue graft 200 includes first and second layers 210, 220. The first layer 210 constitutes a bioremodelable collagenous material, e.g., an ECM layer. The second layer 220 is formed from at least two portions 222, 224 of material that are each different than the material of the first layer 210. Each portion 222, 224 of material may constitute a bioremodelable collagenous material, e.g., an ECM layer, or a substantially remodelable or non-remodelable synthetic or natural material. The portions 222, 224 of material may collectively form a second layer 220 that is the same size as the first layer 210 or a different size. The portions 222, 224 of material of the second layer 220 may abut one another or may be spaced from one another on the first layer 210. As shown in Fig. 6, the portions 222, 224 of material abut one another and are sized such that the first and second layers 210, 220 have the same size and shape. Reinforcing means 22 are provided in the first and second layers 210, 220 in a reinforcing pattern as previously described. As shown in Fig. 6, reinforcing means 22 comprises a plurality of first fibers 40 and a plurality of second fibers 42 stitched in a cross-hatched pattern across and through both layers 210, 220 in order to mitigate tearing and/or improve fixation retention of the first and second layers.

In Fig. 7, the tissue graft 300 includes first, second, and third layers 310, 320, 330. Each of the first layer 310, the second layer 320, and the third layer 330 may constitute a bioremodelable collagenous material, e.g., an ECM layer, or a substantially remodelable or non-remodelable synthetic or natural material. In one instance, the second layer 320 comprises a synthetic layer sandwiched between ECM layers 310, 330. The third layer 320 may be sized and positioned to extend across only one end of the graft 300 overlying the first layer 310 or the second layer 320. In other words, the third layer 330 only partially covers the first layer 310 or the second layer 320, thereby leaving the remainder of the first layer or the second layer exposed. Reinforcing means 22 are provided in the first, second, and third layers 310, 320, 330 in a reinforcing pattern as previously described. As shown in Fig. 7, the reinforcing means 22 comprises a plurality of first fibers 40 and a plurality of second fibers 42 stitched in a cross-hatched pattern across and through all three layers 310, 320, 330 in order to mitigate tearing and/or improve fixation retention of the first, second, and third layers.

In Fig. 8, the tissue graft 400 includes first, second, and third layers 410, 420, 430. Each of the first layer 410, the second layer 420, and the third layer 430 may be a bioremodelable collagenous material, e.g., an ECM layer, or a substantially remodelable or non-remodelable synthetic layer. In one instance, the second layer 420 comprises a synthetic layer sandwiched between ECM layers 410, 430. Unlike the graft 300 of Fig. 7, however, the third layer 420 of the graft 400 of Fig. 8 is sized and positioned to extend across the entire first layer 410 or the second layer 420. Reinforcing means 22 are provided in the first, second, and third layers 410, 420,430 in a reinforcing pattern as previously described. As shown in Fig. 8, the reinforcing means 22 comprises a plurality of first fibers 40 and a plurality of second fibers 42 stitched in a cross-hatched pattern across and through all three layers 410, 420, 430 in order to mitigate tearing and/or improve fixation retention of the first, second, and third layers.

In Figs. 5-8, the layers forming the tissue grafts 100, 200, 300, or 400 may be bonded or secured to one another prior to incorporating the reinforcing means 22 into the layers. The layers may, for example, be laminated, vacuum pressed, lyobonded *(i.e.,* bonding using the lyophlization process), heated, partially cross-linked, etc. prior to incorporating the reinforcing means 22 into the layers. In such a case, the reinforcing means 22 not only improves fixation retention in the grafts 100, 200, 300, or 400 but also helps to minimize relative movement between layers of the grafts. Alternatively, the layers may be simply placed overlying one another prior to incorporating the reinforcing means 22 into the layers. Furthermore, some layers or portions of layers of each tissue graft 100, 200, 300, or 400 may be secured together prior to incorporating the reinforcing means 22 while other portions or layers of the tissue graft remain unsecured to other layers or portions in accordance with the present invention.

In instances where the grafts 100, 200, 300, or 400 include synthetic layers, the synthetic layers help to strengthen and reinforce the graft and facilitate the incorporation of the reinforcing means 22 into the graft. Those having ordinary skill appreciate that although grafts formed from only two and three layers are illustrated, the grafts may be formed from any number of layers of bioremodelable collagenous material or substantially remodelable or non-remodelable synthetic or natural material having reinforcing means incorporated therein in accordance with the present invention.

The tissue graft of the present invention can be used in tissue engineering and musculoskeletal repair, such as rotator cuff repair, but is not restricted to musculoskeletal applications. The graft may be administered to a subject to mechanically and biologically augment the repair. As discussed above, the tissue graft can be used as overlay (over or at the interface of the repair), an interpositional (to fill a space between a tissue and its attachment), or an underlay (under or at the interface of the repair). It will be appreciated that similar methods and materials as described herein could also be adapted to other tendon-to-bone repairs, soft-tissue repairs, such as the repair of lacerated muscles, muscle transfers, spanning a large muscle defect, or use in tendon reinforcement. These applications require secure connections between the graft 10, 100, 200, 300, or 400 and the anatomical site. Fixation techniques to soft tissue using conventional or novel suture methods, or the Pulvertaft weave technique (M. Post, J Shoulder Elbow Surg 1995; 4:1-9) may be utilized in accordance with the present invention.

Fixation techniques to bone using conventional or novel suture methods, anchors, screws, plates, adhesives, staples or tacks may be utilized in accordance with the present application. The graft 10, 100, 200, 300, or 400 may also serve as a delivery platform for the future investigation of any number of biologic strategies aimed to enhance muscular skeletal repair, e.g., rotator cuff healing. Furthermore, the graft 10, 100, 200, 300, or 400 could be effective for other needs in the field of surgical reconstruction including soft-tissue repairs, such as the repair of lacerated muscles, muscle transfers, abdominal wall reconstruction, hernia repair, repair of compartment syndrome releases, tendon reinforcement, or as a bridging material in a subject also be used as a bridging material in a subject, such as where the gap between a tendon and the associated bone is too large to repair conventionally or in abdominal wall procedures with loss of tissue domain..

The following examples are illustrative of the principles and practice of this invention. Numerous additional embodiments within the scope of the invention will become apparent to those skilled in the art.

### Example 1

This example shows stitching dermis and synthetic mesh together using reinforcing fiber improves the mechanical properties of the construct and mitigates tearing and/or improves fixation retention of the layers compared to using either material alone or both materials without stitching them together.

In this example, four groups were investigated:
1. Dermis (native acellular dermis)
2. Mesh (synthetic UHMWPE mesh)
3. Dermis layered against mesh (but not stitched)
4. Dermis layered against mesh and stitched together using 6PLA/2PGA polymer braids.

Uniaxial suture retention tests were used to verify the efficacy of stitching as a method to reinforce the two layers and improve the mechanical properties of the construct by mitigating tearing and/or improving fixation retention of the layers.

All human dermis grafts included allogeneic or xenogeneic dermis.
All 6PLA/2PGA braids used for reinforcing the layers were obtained from Concordia Fibers, Coventry, RI.

### Uniaxial suture retention test

A sample size (n = 3-4) was used in each group. Each specimen consisted of 1.5 cm wide x 4.5 cm long strips of dermis and/or mesh. Dermis and mesh layers were stitched using a 6PLA/2PGA polymer braid (diameter: 400um).

All specimens were hydrated for 30 minutes in saline solution at 37°C. A single simple suture loop of #2 FiberWire was applied using a reverse cutting needle, 7.5mm from one 1.5 cm wide edge; a template was used to assure uniformity in the placement of the sutures. The suture loop was fixed on a hook mounted on the cross head of a MTS 5543 table top system using a 1001b load cell. The other end of the strip was clamped in 7.5mm-deep clamps mounted on the MTS base. The specimen was stretched at 30mm/min and the mode of failure was recorded. The suture retention load was defined as the maximum load attained by the specimen.

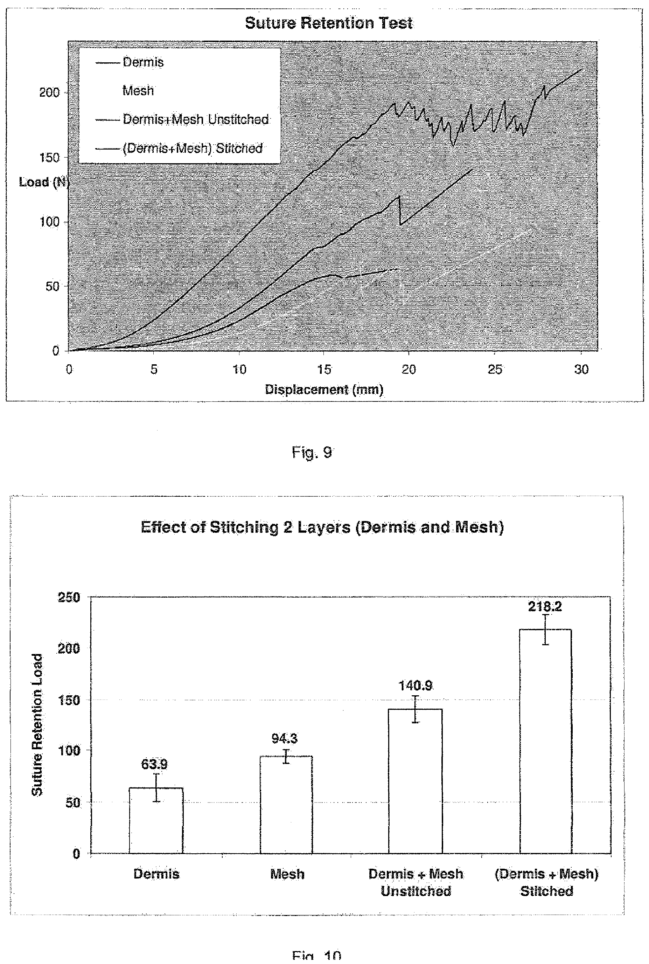

The results of this test are illustrated in Fig. 9, Fig. 10 and Table 1. Fig. 9 (average load-displacement curves) shows that compared to the other three groups, stitching the dermis and synthetic mesh layers together increased toe-stiffness, made the load-displacement curves more linear (increased toe: linear stiffness ratio) and significantly increased suture retention load.

**Table 1. Summary of results from suture retention tests of dermis, synthetic mesh, dermis and mesh layers unstitched and dermis and mesh layers stitched together using 6PLA/2PGA polymer braids. Like letters indicate a significant difference (P<0.05) between groups.**

| | Dermis (n=3) | Mesh (n=4) | Dermis + Mesh Unstitched (n=3) | (Dermis + Mesh) Stitched (n=3) |
|---|---|---|---|---|
| Toe Stiffness (N/mm) | 0.6 ±0.1^{a,b} | 0.3 ±0.01^{a.c.d} | 0.7 ±0.04^{c,e} | 1.7 ±0.1^{b,d,e} |
| Linear Stiffness (N/mm) | ^{a} 8.3 ±2.1 | 9.3 ±1.5 | 12.4 ±3.0 | 13.7 ±0.1^{a} |
| Toe:Linear Stiffness Ratio (%) | 7 ±0.8^{a,b} | 3 ±0.5^{a,c,d} | 6 ±1.2^{c,e} | 12 ±1.0^{b,c,e} |
| Suture Retention Load (N) | ^{a,b,c} 63.9 ±13.3 | 94.3 ±6.3 ^{a,d,e} | 140.9 ±12.9^{b,d,f} | 218.2 ±14.3^{c,e,f} |

These data demonstrate that stitching dermis and synthetic mesh together using reinforcing fiber improves the mechanical properties of the construct and improves suture fixation retention of the layers compared to using either material alone or both materials without stitching them together.

### Example 2

In this example, the biodegradable layer is stitched to a synthetic mesh layer with a stitch pattern known to impart a suture retention load to the construct that is greater than the suture retention load of the dermis alone, the mesh alone, or dermis and mesh that are not stitched together (Example 1). The biodegradable layer could be, for example, ECM derived from human allogeneic or xenogeneic dermis, the synthetic mesh could be made from polypropylene and the stitching fiber could be a PLLA/PGA braid obtained from Concordia Fibers, Coventry, RI. In other embodiments, the synthetic mesh and the stitching fiber could be derived from other synthetic biomaterials with different biodegradation profiles (such as UHMWPE, ePTFE, PLGA and PLLA) or natural biomaterials (such as silk and collagen).

The stitched biodegradable and synthetic mesh layered constructs are hydrated for 30 minutes in saline solution at 37°C. A single simple suture loop of #2 FiberWire is applied to each construct 7.5mm from one end using a reverse cutting needle. Samples are then incubated in 21U/ml collagenase type I solution at 37°C for 6 hours. Uniaxial suture retention tests are performed using a MTS 5543 table top system using a 100lb load cell, where the suture retention load is defined as the maximum load attained by the specimen.

The suture retention properties of the stitched biodegradable and synthetic mesh layered construct are not decreased after collagenase digestion whereas the suture retention properties of the biodegradable layer with stitching but no synthetic layer are decreased modestly and the suture retention properties of the biodegradable layer alone are decreased substantially. This example demonstrates how stitching the biodegradable layer with fiber alone or in combination with a synthetic mesh can potentially substantially maintain and/or minimize the decrease in suture retention properties of the construct while the ECM is remodeling.

While this invention has been particularly shown and described with references to preferred embodiments thereof, it will be understood by those skilled in the art that various changes in form and details may be made therein without departing from the scope of the invention encompassed by the appended claims.

## Claims

1. A biocompatible tissue graft comprising:
a first layer of a bioremodelable collageneous material comprising extracellular matrix;
a second layer of biocompatible synthetic remodelable or substantially remodelable material attached to the first layer, wherein the second layer comprises a mesh of synthetic material; and
a plurality of first fibers and a plurality of second fibers stitched in a cross-hatched reinforcing pattern in the first layer and the second layer, to mitigate tearing and/or improve fixation retention of the layers, and substantially maintain the improved properties while one or more of the layers is remodeling;
wherein the ends of the first fibers and the ends of the second fibers are stitched together to form a continuous stitch construction.

2. The tissue graft of claim 1, wherein the fiber comprises a natural or synthetic material.

3. The tissue graft of claim 2, wherein the fiber is selected from the group consisting of collagen, silk, sericin free silk, modified silk fibroins, polyesters optionally selected from PGA, PLA, polylactic-co-glycolic acid (PLGA), polyethyleneglycol (PEG), polyhydroxyalkanoates (PHA), polyethylene terephthalate (PET), polyethylene (PE), ultra-high molecular weight polyethylene (UHMWPE), blends thereof, and copolymers thereof.

4. The tissue graft of claim 2, wherein at least one of the first layer, the second layer, and the fiber is modified to improve adhesion between the layers and the fiber.

5. The tissue graft of claim 1, wherein the extracellular matrix is decellularized.

6. The tissue graft of claim 1, wherein at least one of the first layer or the second layer further comprises at least one differentiated or progenitor cell.

7. The tissue graft of claim 1, wherein at least one of the first layer or the second layer further comprises at least one biologically active molecule selected from the group consisting of drugs, sclerosing agents, enzymes, hormones, cytokines, colony-stimulating factors, vaccine antigens, antibodies, clotting factors, angiogenesis factors, regulatory proteins, transcription factors, receptors, structural proteins, nucleic acid therapeutic agents, and combinations thereof.

8. The tissue graft of claim 1, wherein the tissue graft comprises a first side and a second side connecting a top surface to a bottom surface of the tissue graft; and the plurality of first fibers and the plurality of second fibers are stitched in a cross-hatched pattern across the graft and between the first and second sides and the front and rear surfaces.

9. The tissue graft of claim 8, wherein the second fiber is disposed in an overlying fashion relative to the first fiber such that the first fiber is disposed between a top surface of the graft and the second fiber.

10. The tissue graft of claim 8, wherein the second fiber is disposed between a top surface of the graft and the first fiber.

11. The tissue graft of claim 8, wherein the first fiber extends from the first side to the second side of the graft, and the second fiber extends from the front surface to the rear surface of the graft.

12. The tissue graft of claim 1, further comprising a third layer.

13. The tissue graft of claim 12, wherein the first layer, the second layer, and the third layer are stitched together with the fiber.

## Patentansprüche

1. Biokompatibles Gewebetransplantat, das Folgendes umfasst:
eine erste Schicht eines bioremodellierbaren kollagenen Materials, die extrazelluläre Matrix umfasst;
eine zweite Schicht von biokompatiblem synthetischem remodellierbarem oder im Wesentlichen remodellierbarem Material, die an der ersten Schicht angebracht ist, wobei die zweite Schicht ein Netz von synthetischem Material umfasst; und
eine Vielzahl von ersten Fasern und eine Vielzahl von zweiten Fasern, die in einem verstärkenden Gittermuster in der ersten Schicht und der zweiten Schicht vernäht sind, um ein Reißen zu vermindern und/oder die Erhaltung der Befestigung der Schichten zu verbessern und im Wesentlichen die verbesserten Eigenschaften zu erhalten, während eine oder mehrere der Schichten remodelliert werden;
wobei die Enden der ersten Fasern und die Enden der zweiten Fasern zusammen vernäht sind, um eine fortlaufende Nahtkonstruktion zu bilden.

2. Gewebetransplantat nach Anspruch 1, wobei die Faser ein natürliches oder synthetisches Material umfasst.

3. Gewebetransplantat nach Anspruch 2, wobei die Faser aus der Gruppe ausgewählt ist, die aus Folgenden besteht: Kollagen, Seide, Sericin-freier Seide, modifizierten Seidenfibroinen, Polyestern, die optional aus Folgenden ausgewählt sind: PGA, PLA, Poly(milchsäure-co-glycolsäure) (PLGA), Polyethylenglycol (PEG), Polyhydroxyalkanoaten (PHA), Polyethylenterephthalat (PET), Polyethylen (PE), ultrahochmolekularem Polyethylen (UHMWPE), Gemischen davon und Copolymeren davon.

4. Gewebetransplantat nach Anspruch 2, wobei mindestens eines von der ersten Schicht, der zweiten Schicht und der Faser modifiziert ist, um die Haftung zwischen den Schichten und der Faser zu verbessern.

5. Gewebetransplantat nach Anspruch 1, wobei die extrazelluläre Matrix dezellularisiert ist.

6. Gewebetransplantat nach Anspruch 1, wobei mindestens eine von der ersten Schicht oder der zweiten Schicht weiter mindestens eine differenzierte oder Progenitorzelle umfasst.

7. Gewebetransplantat nach Anspruch 1, wobei mindestens eine von der ersten Schicht oder der zweiten Schicht weiter mindestens ein biologisch aktives Molekül umfasst, das aus der Gruppe ausgewählt ist, die aus Folgenden besteht: Arzneimitteln, Sklerosierungsmitteln, Enzymen, Hormonen, Zytokinen, koloniestimulierenden Faktoren, Impfstoffantigenen, Antikörpern, Gerinnungsfaktoren, Angiogenesefaktoren, regulatorischen Proteinen, Transkriptionsfaktoren, Rezeptoren, Strukturproteinen, Nukleinsäure-Therapeutika und Kombinationen davon.

8. Gewebetransplantat nach Anspruch 1, wobei das Gewebetransplantat eine erste Seite und eine zweite Seite umfasst, die eine obere Oberfläche mit einer unteren Oberfläche des Gewebetransplantats verbinden, und die Vielzahl von ersten Fasern und die Vielzahl von zweiten Fasern in einem Gittermuster über das Transplantat und zwischen der ersten und zweiten Seite und der vorderen und hinteren Oberfläche vernäht sind.

9. Gewebetransplantat nach Anspruch 8, wobei die zweite Faser in einer übereinanderliegenden Weise in Bezug auf die erste Faser angeordnet ist, sodass die erste Faser zwischen einer oberen Oberfläche des Transplantats und der zweiten Faser angeordnet ist.

10. Gewebetransplantat nach Anspruch 8, wobei die zweite Faser zwischen einer oberen Oberfläche des Transplantats und der ersten Faser angeordnet ist.

11. Gewebetransplantat nach Anspruch 8, wobei sich die erste Faser von der ersten Seite zu der zweiten Seite des Transplantats erstreckt und sich die zweite Faser von der vorderen Oberfläche zu der hinteren Oberfläche des Transplantats erstreckt.

12. Gewebetransplantat nach Anspruch 1, das weiter eine dritte Schicht umfasst.

13. Gewebetransplantat nach Anspruch 12, wobei die erste Schicht, die zweite Schicht und die dritte Schicht mit der Faser zusammen vernäht sind.

## Revendications

1. Greffon tissulaire biocompatible comprenant :
une première couche d'un matériau collagénique bioremodelable comprenant une matrice extracellulaire ;
une deuxième couche de matériau synthétique biocompatible remodelable ou substantiellement remodelable attaché à la première couche, dans lequel la deuxième couche comprend une maille de matériau synthétique ; et
une pluralité de premières fibres et une pluralité de deuxièmes fibres cousues selon un motif de renforcement quadrillé dans la première couche et la deuxième couche, pour atténuer le déchirement et/ou améliorer la tenue de fixation des couches, et substantiellement maintenir les propriétés améliorées pendant le remodelage d'une ou de plusieurs des couches ;
dans lequel les extrémités des premières fibres et les extrémités des deuxièmes fibres sont cousues ensemble pour former une construction continue de piqûres.

2. Greffon tissulaire selon la revendication 1, dans lequel la fibre comprend un matériau naturel ou synthétique.

3. Greffon tissulaire selon la revendication 2, dans lequel la fibre est choisie parmi le groupe constitué par le collagène, la soie, la soie exempte de séricine, des fibroïnes de soie modifiées, des polyesters choisis facultativement parmi le PGA, le PLA, le poly(acide lactique-co-glycolique) (PLGA), le polyéthylène glycol (PEG), des polyhydroxyalcanoates (PHA), le poly(téréphtalate de d'éthylène) (PET), le polyéthylène (PE), le polyéthylène à très haut poids moléculaire (UHMWPE), des mélanges de ceux-ci, et des copolymères de ceux-ci.

4. Greffon tissulaire selon la revendication 2, dans lequel au moins l'une de la première couche, de la deuxième couche et de la fibre est modifiée pour améliorer l'adhésion entre les couches et la fibre.

5. Greffon tissulaire selon la revendication 1, dans lequel la matrice extracellulaire est décellularisée.

6. Greffon tissulaire selon la revendication 1, dans lequel au moins l'une de la première couche ou de la deuxième couche comprend en outre au moins une cellule différenciée ou progénitrice.

7. Greffon tissulaire selon la revendication 1, dans lequel au moins l'une de la première couche ou de la deuxième couche comprend en outre au moins une molécule biologiquement active choisie parmi le groupe constitué par des médicaments, des agents sclérosants, des enzymes, des hormones, des cytokines, des facteurs de stimulation des colonies, des antigènes vaccinaux, des anticorps, des facteurs de coagulation, des facteurs angiogéniques, des protéines régulatrices, des facteurs de transcription, des récepteurs, des protéines structurales, des agents thérapeutiques d'acide nucléique, et des combinaisons de ceux-ci.

8. Greffon tissulaire selon la revendication 1, dans lequel le greffon tissulaire comprend un premier côté et un deuxième côté raccordant une surface supérieure à une surface inférieure du greffon tissulaire ; et la pluralité de premières fibres et la pluralité de deuxièmes fibres sont cousues selon un motif quadrillé sur le greffon et entre les premier et deuxième côtés et les surfaces antérieure et postérieure.

9. Greffon tissulaire selon la revendication 8, dans lequel la deuxième fibre est disposée de manière à recouvrir la première fibre de sorte que la première fibre est disposée entre une surface supérieure du greffon et la deuxième fibre.

10. Greffon tissulaire selon la revendication 8, dans lequel la deuxième fibre est disposée entre une surface supérieure du greffon et la première fibre.

11. Greffon tissulaire selon la revendication 8, dans lequel la première fibre s'étend du premier côté au deuxième côté du greffon, et la deuxième fibre s'étend de la surface antérieure à la surface postérieure du greffon.

12. Greffon tissulaire selon la revendication 1, comprenant en outre une troisième couche.

13. Greffon tissulaire selon la revendication 12, dans lequel la première couche, la deuxième couche, et la troisième couche sont cousues ensemble avec la fibre.
